(19) Europäisches Patentamt / European Patent Office / Office européen des brevets

(11) **EP 4 432 219 B1**

(12) **EUROPEAN PATENT SPECIFICATION**

(45) Date of publication and mention
of the grant of the patent:
**25.03.2026 Bulletin 2026/13**

(21) Application number: **22900329.8**

(22) Date of filing: **23.11.2022**

(51) International Patent Classification (IPC):
*G16H 50/20* (2018.01)   *G06V 10/82* (2022.01)
*G06V 10/80* (2022.01)   *G06T 7/00* (2017.01)
*G06N 3/08* (2023.01)   *G06N 3/045* (2023.01)
*G06F 18/25* (2023.01)   *G06F 18/214* (2023.01)
*G16H 20/00* (2018.01)   *G16H 30/40* (2018.01)
*G16H 50/70* (2018.01)   *G16H 50/30* (2018.01)
*G06N 3/0464* (2023.01)   *G06N 3/084* (2023.01)
*G06N 3/09* (2023.01)

(52) Cooperative Patent Classification (CPC):
G06T 7/0012; G06F 18/214; G06F 18/253;
G06N 3/045; G06N 3/0464; G06N 3/084;
G06N 3/09; G06V 10/806; G06V 10/82;
G16H 20/00; G16H 30/40; G16H 50/20;
G16H 50/30; G16H 50/70; G06V 2201/03

(86) International application number:
**PCT/CN2022/133614**

(87) International publication number:
**WO 2023/098524 (08.06.2023 Gazette 2023/23)**

(54) **MULTI-MODAL MEDICAL DATA FUSION EVALUATION METHOD AND APPARATUS, DEVICE, AND STORAGE MEDIUM**

VERFAHREN UND VORRICHTUNG ZUR MULTIMODALEN MEDIZINISCHEN DATENFUSIONSBEURTEILUNG, VORRICHTUNG UND SPEICHERMEDIUM

PROCÉDÉ ET APPAREIL D'ÉVALUATION DE FUSION DE DONNÉES MÉDICALES MULTIMODALES, DISPOSITIF ET SUPPORT DE STOCKAGE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.12.2021 CN 202111454543**

(43) Date of publication of application:
**18.09.2024 Bulletin 2024/38**

(73) Proprietor: **Tianjin Yujin Artificial Intelligence Medical**
**Technology Co., Ltd**
**Tianjin 300457 (CN)**

(72) Inventor: **WANG, Yufeng**
**Tianjin 300457 (CN)**

(74) Representative: **Cabinet Chaillot**
**16/20, avenue de l'Agent Sarre**
**B.P. 74**
**92703 Colombes Cedex (FR)**

(56) References cited:
**CN-A- 111 079 864      CN-A- 111 931 795**
**CN-A- 113 657 503      CN-A- 113 870 259**
**US-A1- 2018 367 871**

• **DAI YIN ET AL: "TransMed: Transformers Advance Multi-Modal Medical Image Classification", DIAGNOSTICS, 31 July 2021 (2021-07-31), CH, pages 1 - 15, XP093235735, ISSN: 2075-4418, Retrieved from the Internet <URL:https://pmc.ncbi.nlm.nih.gov/articles/ PMC8391808/pdf/diagnostics-11-01384.pdf> [retrieved on 20241219], DOI: 10.3390/ diagnostics11081384**

- ASHISH VASWAN ET AL: "Attention Is All You Need", 31ST CONFERENCE ON NEURAL INFORMATION PROCESSING SYSTEMS (NIPS 2017), 12 June 2017 (2017-06-12), XP055506908, Retrieved from the Internet <URL:https://arxiv.org/pdf/1706.03762.pdf> [retrieved on 20180913]
- HO DANLIANG HO DANLIANG@U NUS EDU ET AL: "Predictive models for colorectal cancer recurrence using multi-modal healthcare data", ACM SYMPOSIUM ON EYE TRACKING RESEARCH AND APPLICATIONS, ACMPUB27, NEW YORK, NY, USA, 8 April 2021 (2021-04-08), pages 204 - 213, XP058533582, ISBN: 978-1-4503-8365-3, DOI: 10.1145/3450439.3451868

**Description**

TECHNICAL FIELD

The present application relates to the technical field of medical treatment, and in particular, to an evaluation method and device for multimodal medical data fusion, as well as a device and a storage medium.

BACKGROUND

Rectal cancer is one of the major cancers threatening the life and health of Chinese residents, causing a serious social burden. The main treatment methods of rectal cancer include surgery, radiotherapy, chemotherapy, targeted therapy and other comprehensive treatment methods. Although there are standardized comprehensive treatment methods, damage caused by tumor or surgery in patients with low rectal cancer may lead to anal function damage, anal loss and colostomy, which seriously affect the survival and treatment of the patients. Many patients with locally advanced rectal cancer are not suitable for surgical treatment because primary surgery cannot achieve the goal of radical cure. At present, standard treatment for locally advanced rectal cancer ($\geq$cT3 or N+) is comprehensive treatment of neoadjuvant chemoradiotherapy combined with total mesorectal excision and adjuvant chemotherapy. Neoadjuvant therapy may effectively realize the tumor down-staging, improve resection rate and anus preservation rate. The neoadjuvant therapy also provides a better choice for preserving organ function of patients with low rectal cancer. Evaluation of the efficacy of the neoadjuvant therapy for rectal cancer, namely, whether clinical remission is achieved after treatment and the probability of achieving pathological remission, is the key link to make clinical decisions and evaluate the prognosis of patients.

In the evaluation of the efficacy of the neoadjuvant therapy for rectal cancer at the present stage, most clinical guidelines and expert consensus suggest that the multimodal data such as endoscopy, digital rectal examination, rectal magnetic resonance imaging, serum tumor marker level and chest, abdomen and pelvis enhanced CT should be used to comprehensively determine whether a patient has achieved clinical remission or is close to clinical remission. The evaluation of the efficacy of the neoadjuvant therapy for rectal cancer depends on a multidisciplinary tumor diagnosis and treatment team with experienced experts in surgery, internal medicine, radiotherapy, imaging, digestive endoscopy, pathology and other departments. Due to the lack of experts in certain professional directions, many medical institutions cannot carry out the neoadjuvant therapy for rectal cancer well. The dependence on expert experience also leads to judgment error and inconsistent decision criteria of the evaluation of the efficacy of the neoadjuvant therapy for rectal cancer due to human factors. A tool and a method that can synthesize the multimodal medical data and objectively and consistently evaluate the efficacy of the neoadjuvant therapy for rectal cancer are urgently needed. HO DANLIANG HO DANLIANG@U NUS EDU ET AL: "Predictive models for colorectal cancer recurrence using multi-modal healthcare data", ACM SYMPOSIUM ON EYE TRACKING RESEARCH AND APPLICATIONS, ACMPUB27, NEW YORK, NY, USA, 8 April 2021 (2021-04-08), pages 204-213. Retrieved from DOI: 10.1145/3450439.3451868 discusses predictive models for colorectal cancer reccurence using multi-modal healthcare data.

SUMMARY

In order to have a basic understanding of some aspects of disclosed examples, a brief summary is given below. The summary is not a general comment, nor is it intended to determine key/important constituent elements, but rather serves as a prelude to the following detailed description.

An example of the present disclosure provides an evaluation method and device for multimodal medical data fusion, as well as a device and a storage medium to solve the technical problem that clinicians are difficult to accurately evaluate patients' disease remission by manual means, resulting in high medical risk of the patients in the related art.

In some examples, an example of the present disclosure provides an evaluation method for multimodal medical data fusion, including:

acquiring to-be-evaluated medical data in multiple modes of a target object;
performing feature extraction on to-be-evaluated medical data in each mode to obtain a plurality of feature vectors;
fusing the a plurality of feature vectors to obtain a fusion feature vector; and
inputting the fusion feature vector into a pre-trained multimodal fusion evaluation model to obtain an evaluation result of the to-be-evaluated medical data in multiple modes output by the pre-trained multimodal fusion evaluation mode, wherein
the inputting the fusion feature vector into a pre-trained multimodal fusion evaluation model to obtain an evaluation result of the to-be-evaluated medical data in multiple modes output by the pre-trained multimodal fusion evaluation model includes:

splicing each feature vector in the fusion feature vector horizontally to obtain a first matrix W(In) of feature vector, and coding position of the first matrix W(In) of feature vector by a first function to obtain a second matrix W(P) of feature vector. The formula is as follows:

$$p(t) = \begin{cases} sin\left(\dfrac{pos}{10000^{2i/d}}\right), & i\ \%\ 2 = 0 \\ cos\left(\dfrac{pos}{10000^{2i/d}}\right), & i\ \%\ 2 = 1 \end{cases}$$

where, t represents a sub-vector in the first matrix W(In) of feature vector; p(t) represents a coding result corresponding to the t value; pos indicates which feature vector the vector t belongs to; i represents a sequence number of the vector t in the first matrix W(In) of feature vector; and d represents the number of horizontal dimensions of the first matrix W(In) of feature vector;

inputting the second matrix W(P) of feature vector into a second function, and calculating a high-dimensional feature representation matrix W(M) on a subspace. The formula is as follows:

$$W(M) = \mathrm{Concat}\left(F(1), F(2), \ldots, F(i)\right) \cdot W^0;$$

$$F(i) = \mathrm{softmax}\left(\frac{Q(x)\ K^T(x)}{\sqrt{d(x)}}\right) \cdot V$$

where, a CONCAT function represents the second function, and F(1), F(2)... F(i) represents the ith feature sub-vector in the second matrix W(P) of feature vector is conducted the formula F calculation; $W^0$ represents transposition of the first matrix W(In) of feature vector;

x in F(i) represents the ith feature sub-vector in the input second matrix W(P) of feature vector; Q, K and V represent the linear sensing layer of parameter n of the hidden layer of the multimodal fusion evaluation model; and Q (x) represents linear regression of x; and

coding the feature vector of each image through an encoder of the multimodal fusion evaluation model, inputting output W(Out) of the encoder to a linear regression layer, converting W(Out) to a low-dimensional feature representation matrix through the linear regression layer, and finally outputting an evaluation result through operation of a softmax function; the final result is an evaluation result of complete or incomplete disease remission of the target object, as well as probability corresponding to the evaluation result; wherein

the acquiring the to-be-evaluated medical data in multiple modes of the target object includes at least three of the following:

acquiring a rectal cancer image data set of the target object as first modal data, where the rectal cancer image data set at least includes a macro-view image, a near-view image and a micro-view image determined according to a tumor region or a regressing tumor region;

acquiring a rectal cancer magnetic resonance image data set of the target object as second modal data, where the rectal cancer magnetic resonance image data set includes initial rectal cancer magnetic resonance image data and target rectal cancer magnetic resonance image data; marking a tumor region or a regressing tumor region in the initial rectal cancer magnetic resonance image data and the target rectal cancer magnetic resonance image data respectively to obtain a plurality of slice images containing the tumor region or the regressing tumor region;

acquiring an initial clinical data set and a target clinical data set of the target object as a third modal data, where the initial clinical data set and the target clinical data set at least include personal information and case information of the target object; and

acquiring initial tumor marker information, target tumor marker information, initial blood information and target blood information of the target object as fourth modal data.

In some examples, the performing feature extraction on to-be-evaluated medical data in each mode to obtain a plurality of feature vectors includes;

inputting the first modal data and the second modal data to a pre-trained neural network model respectively;

performing matrix connection on medical images in the first modal data and the second modal data respectively through a hard-wired layer of the neural network model;

performing convolution calculation and maximum pooling operation on a medical image after the matrix connection through alpha 3D convolution modules of the neural network model to extract high-dimensional feature maps; and

converting a high-dimensional feature map extracted from the last 3D convolution kernel into a one-dimensional feature vector through beta up-sampling modules and a full-connection layer of the neural network model to obtain the first feature vector and a second feature vector respectively.

In some examples, the performing feature extraction on to-be-evaluated medical data in each mode to obtain a plurality of feature vectors includes;

mapping text description features in the third modal data and the fourth modal data into corresponding numerical features; and

mapping the numerical features into a two-dimensional matrix to obtain a third feature vector and a fourth feature vector respectively.

In some examples, a training process of the neural network model includes:

inputting acquired preset to-be-evaluated medical data as a training sample into a corresponding initial neural network model to enable the initial neural network model to output a corresponding initial feature vector;

if the initial feature vector meets preset requirements, determining the initial neural network model to be successfully trained to obtain the pre-trained neural network model;

if the initial feature vector does not meet the preset requirements, continuing to train the initial neural network model by adjusting a loss parameter in the initial neural network model until the loss parameter is fitted to reach a preset loss parameter threshold so as obtain the pre-trained neural network model.

In some examples, a cross-entropy loss function is used for parameter back-propagation and update during training of the multimodal fusion evaluation model until the cross-entropy loss function is fitted.

In some examples, an example of the present disclosure provides an evaluation device for multimodal medical data fusion, including:

a medical data acquisition module, configured to acquire to-be-evaluated medical data in multiple modes of a target object;

an feature vector extraction module, configured to perform feature extraction on to-be-evaluated medical data in each mode to obtain a plurality of feature vectors;

an feature vector fusion module, configured to fuse the a plurality of feature vectors to obtain a fusion feature vector; and

a multimodal fusion evaluation module, configured to input the fusion feature vector into a pre-trained multimodal fusion evaluation model to obtain an evaluation result of the to-be-evaluated medical data in multiple modes output by the pre-trained multimodal fusion evaluation model.

In some examples, an example of the present disclosure provides an electronic device, including a processor, a communication interface, a memory and a communication bus, where the processor, the communication interface and the memory complete communication with each other through the communication bus;

a memory, configured to store a computer program; and

a processor, configured to implement the method steps when executing the program stored on the memory.

In some examples, an example of the present disclosure provides a computer-readable storage medium, where a computer program is stored, where method steps are implemented when the computer program is executed by a processor.

An evaluation method and device for multimodal medical data fusion, as well as a device and a storage medium provided by examples of the present disclosure may achieve the following technical effects:

The examples of the present disclosure perform feature extraction on multimodal medical data on the basis of artificial intelligence to obtain a plurality of feature vectors, perform fusion on the obtained a plurality of feature vectors to obtain a fusion feature vector, use a trained multimodal fusion evaluation model to predict and evaluate remission degree of a target object on the basis of the fusion feature vector, and may assist in accurate evaluation of the disease remission degree of the target object at the pathological level after treatment, thus improving judgment accuracy and reducing medical risks of the target object.

BRIEF DESCRIPTION OF DRAWINGS

At least one example is illustratively specified by the drawings, while the illustrative specification and drawings do not constitute a limitation of the example. Elements with the same reference numerals in the drawings are shown as similar elements, and the drawings do not constitute a scale limitation, and where:

FIG. 1 is a schematic flowchart of an evaluation method for multimodal medical data fusion according to an example of the present disclosure;
FIG. 2 is a schematic diagram of feature extraction and data evaluation of multimodal medical data according to an example of the present disclosure;
FIG. 3 is a schematic structural diagram of an evaluation device for multimodal medical data fusion according to an example of the present disclosure;
FIG. 4 is a schematic structural diagram of an electronic device according to an example of the present disclosure.

DETAILED DESCRIPTION OF THE EMBODIMENTS

In order to understand the features and technical content of examples of the present disclosure in more detail, the implementation of the examples of the present disclosure is described in detail below in combination with the drawings. The attached drawings are for reference only, not for limiting the examples of the present disclosure. In the following technical description, for convenience of explanation, a number of details are provided for a full understanding of the disclosed examples. However, without these details, at least one example may still be implemented. In other cases, in order to simplify the drawings, familiar structures and devices may be simplified.

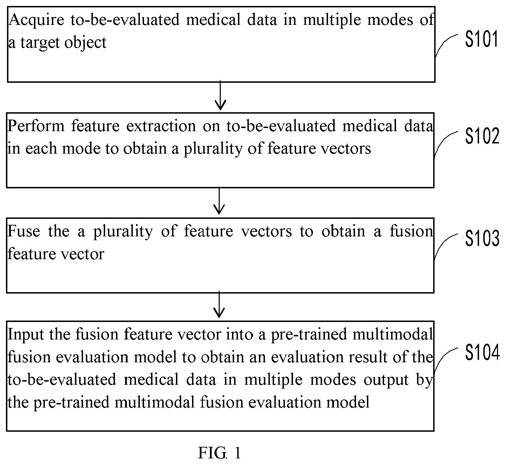

An example of the present disclosure provides an evaluation method for multimodal medical data fusion, as shown in FIG 1, the method including the following steps:

S101, acquiring to-be-evaluated medical data in multiple modes of a target object;
S102, performing feature extraction on to-be-evaluated medical data in each mode respectively to obtain a plurality of feature vectors;
S103, fusing the a plurality of feature vectors to obtain a fusion feature vector; and
S104, inputting the fusion feature vector into a pre-trained multimodal fusion evaluation model to obtain an evaluation result of the to-be-evaluated medical data in multiple modes output by the pre-trained multimodal fusion evaluation model.

In some examples, the acquiring the to-be-evaluated medical data in multiple modes of the target object includes at least three of the following:

acquiring a rectal cancer image data set of the target object as first modal data though an endoscope, where the rectal cancer image data set at least includes a macro-view image (usually 1), a near-view image (usually 1) and a micro-view image (usually 2) determined according to a tumor region or a regressing tumor region; the macro-view image refers to a panoramic image of an area within a first preset distance range from the tumor region or the regressing tumor region and facing the center of the intestinal cavity, for example, a panoramic image of an area 0.8mm-20mm away from the "tumor region" or the "regressing tumor region" and facing the center of the intestinal cavity is taken as the macro-view image; a near-view image refers to an image with the longest boundary of the tumor region or the regressing tumor region less than a preset zoom ratio of a view field boundary, for example, an image taken when the longest boundary of the "tumor region" or the "regressing tumor region" is less than 10% of the view field boundary is regarded as the near-view image; and the micro-view image refers to a local image that is within a preset threshold range (for example, within 0.8mm) from the tumor region or the regressing tumor region and faces the tumor surface.
acquiring a rectal cancer magnetic resonance image data set of the target object as second modal data, where the rectal cancer magnetic resonance image data set includes initial rectal cancer magnetic resonance image data and target rectal cancer magnetic resonance image data; marking a tumor region or a regressing tumor region in the initial rectal cancer magnetic resonance image data and the target rectal cancer magnetic resonance image data respectively through automatic or manual annotation to obtain a plurality of slice images containing the tumor region or the regressing tumor region. Where, the initial rectal cancer magnetic resonance image data may be data of the target object before treatment, and the target rectal cancer magnetic resonance image data may be data of the target object after treatment.
acquiring an initial clinical data set and a target clinical data set of the target object as a third modal data, where the initial clinical data set and the target clinical data set at least include personal information and case information of the target object. The initial clinical data set may be data of the target object before treatment, and the target clinical data set may be data of the target object after treatment. Personal information of the target object may include but not limited to age, height, weight and other information. Case information of the target object may include but not limited to family history of malignant tumor, personal history of tumor, treatment plan, tumor location, tumor differentiation degree, T staging before treatment, N staging before treatment, depth of tumor invasion, distance from tumor to anal edge and other information.

acquiring initial tumor marker information, target tumor marker information, initial blood information and target blood information of the target object as fourth modal data. Where, the initial tumor marker information and the initial blood information may be data of the target object before treatment, and the target tumor marker information and the target blood information may be data of the target object after treatment. Optionally, the initial tumor marker information and the target tumor marker information may include but not limited to data of carbohydrate antigen 125 (CA125), carbohydrate antigen 153 (CA153), carbohydrate antigen 199 (CA199), carcinoembryonic antigen (CEA) and alpha-fetoprotein (AFP); and the initial blood information and the target blood information may include but not limited to blood routine data such as red blood cells, hemoglobin, platelets, platelet volume, white blood cells, neutrophils, lymphocytes, monocytes, C-reactive protein, hypersensitive C-reactive protein, total protein, albumin and prealbumin.

In some examples, the performing feature extraction on to-be-evaluated medical data in each mode to obtain a plurality of feature vectors includes;

inputting first modal data to a pre-trained first neural network model;
performing matrix connection on a macro-view image, a near-view image and a micro-view image through a hard-wired layer of the first neural network model;
performing convolution calculation and maximum pooling operation on the macro-view image, the near-view image and the micro-view image after the matrix connection through alpha 3D convolution modules of the first neural network model to extract high-dimensional feature maps; and
converting a high-dimensional feature map extracted from the last 3D convolution kernel into a one-dimensional feature vector through beta up-sampling modules and a full-connection layer of the first neural network model to obtain a first feature vector, where alpha may be 7 and beta may be 5.

In some examples, the performing feature extraction on to-be-evaluated medical data in each mode to obtain a plurality of feature vectors includes;

inputting second modal data to a pre-trained second neural network model;
performing matrix connection on a plurality of slice images containing a tumor region or a regressing tumor region marked in the second modal data through a hard-wired of the second neural network model;
performing convolution calculation and maximum pooling operation on a slice image after the matrix connection through alpha 3D convolution modules of the second neural network model to extract high-dimensional feature maps; and
converting a high-dimensional feature map extracted from the last 3D convolution kernel into a one-dimensional feature vector through beta up-sampling modules and a full-connection layer of the second neural network model to obtain a second feature vector, where alpha may be 5 and beta may be 3.

In some examples, a training process of the first neural network model and the second neural network model includes:

inputting acquired preset to-be-evaluated medical data as a training sample into a corresponding initial neural network model to enable the initial neural network model to output a corresponding initial feature vector;
if the initial feature vector meets preset requirements, determining the initial neural network model to be successfully trained to obtain the pre-trained neural network model;
if the initial feature vector does not meet the preset requirements, continuing to train the initial neural network model by adjusting a loss parameter in the initial neural network model until the loss parameter is fitted to reach a preset loss parameter threshold so as obtain the pre-trained neural network model.

Optionally, the first neural network model and the second neural network model may adopt a 3D convolution network (3DCNN), which is not limited by the examples of the present disclosure.

In some examples, the performing feature extraction on to-be-evaluated medical data in each mode to obtain a plurality of feature vectors includes;

mapping text description features in third modal data and fourth modal data into corresponding numerical features;

and

mapping numerical features into a two-dimensional matrix to obtain a third feature vector and a fourth feature vector respectively.

Optionally, in the process of feature extraction for the third modal data, if the target object has no family history of malignant tumor, it is mapped to the number 0; If the target object has a family history of malignant tumor, it is mapped to the number 1; similarly, other text description features are mapped to corresponding numerical features as follows:

Personal history of tumor (no 0, yes 1), recurrent tumor (yes 1, no 0), neoadjuvant chemotherapy (yes 1, no 0), neoadjuvant radiotherapy (yes 1, no 0), treatment plan (single 1, double 2, triple 3), tumor location (upper rectal 1, middle rectal 2, lower rectal 3), tumor differentiation degree (high differentiation degree 1, medium differentiation degree 2, low differentiation degree 3) and size (accounting for 1/3 of intestinal circumference 0, accounting for 2/3 of intestinal circumference 1, accounting for whole of intestinal circumference 2).

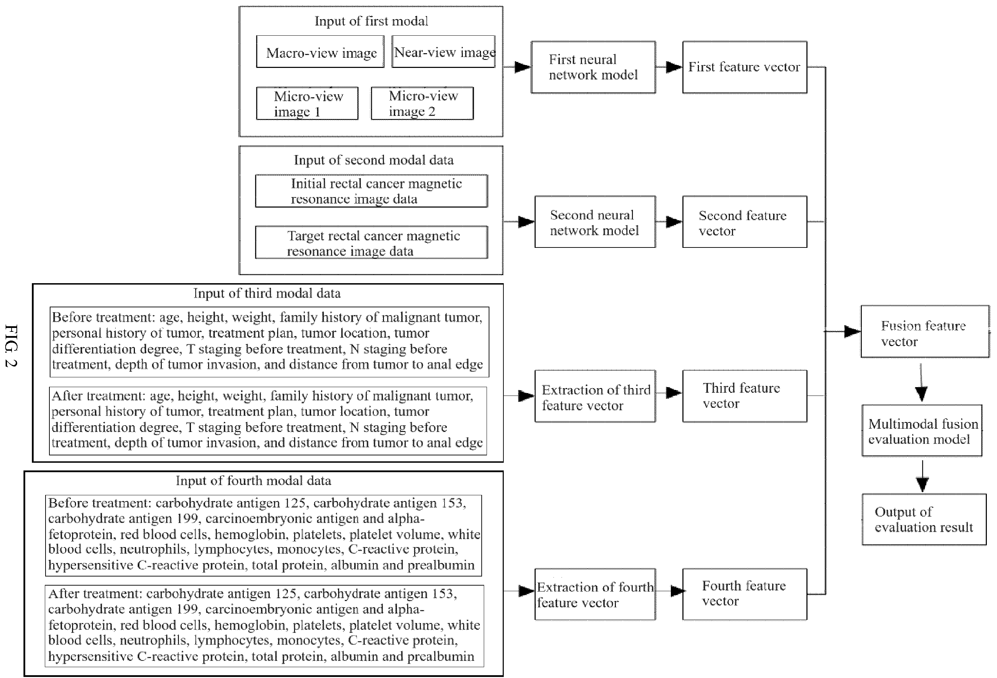

As shown in FIG 2, in some examples, the inputting a fusion feature vector into a pre-trained multimodal fusion evaluation model to obtain an evaluation result of to-be-evaluated medical data in multiple modes output by the pre-trained multimodal fusion evaluation model includes:

splicing each feature vector in the fusion feature vector horizontally to obtain a first matrix W(In) of feature vector, and coding position of the first matrix W(In) of feature vector by a first function to obtain a second matrix W(P) of feature vector. The formula is as follows:

$$p(t) = \begin{cases} sin\left(\dfrac{pos}{10000^{2i/d}}\right), & i \% 2 = 0 \\ cos\left(\dfrac{pos}{10000^{2i/d}}\right), & i \% 2 = 1 \end{cases}$$

where, t represents a sub-vector in the first matrix W(In) of feature vector; p(t) represents a coding result corresponding to the t value; pos indicates which feature vector the vector t belongs to; i represents a sequence number of the vector t in the first matrix W(In) of feature vector; and d represents the number of horizontal dimensions of the first matrix W(In) of feature vector;

inputting the second matrix W(P) of feature vector into a second function, and calculating and obtaining a high-dimensional feature representation matrix W(M) on a subspace. The formula is as follows:

$$W(M) = Concat\left(F(1), F(2), \ldots, F(i)\right) \cdot W^0;$$

$$F(i) = softmax\left(\dfrac{Q(x)\ K^T(x)}{\sqrt{d(x)}}\right) \cdot V$$

where, a CONCAT function represents the second function, and F(1), F(2)... F(i) represents the ith feature sub-vector in the second matrix W(P) of feature vector is conducted the formula F calculation; $W^0$ represents transposition of the first matrix W(In) of feature vector;

x in F(i) represents the ith feature sub-vector in the input second matrix W(P) of feature vector; Q, K and V represent the linear sensing layer of parameter n of the hidden layer of the multimodal fusion evaluation model; and Q (x) represents linear regression of x; and

coding feature vectors of each image through an encoder of the multimodal fusion evaluation model, inputting output W(Out) of the encoder to a linear regression layer, converting W(Out) to a low-dimensional feature representation matrix through the linear regression layer, and finally outputting an evaluation result through operation of a softmax function. A decision is completed by inputting first, second, third and fourth feature vectors to the pre-trained multimodal fusion evaluation model, and a final result is an evaluation result of complete or incomplete disease remission of the target object, as well as probability corresponding to the evaluation result, such as probability of complete remission and probability of incomplete remission.

Optionally, a cross-entropy loss function is used for parameter back-propagation and update during training of the multimodal fusion evaluation model until the cross-entropy loss function is fitted.

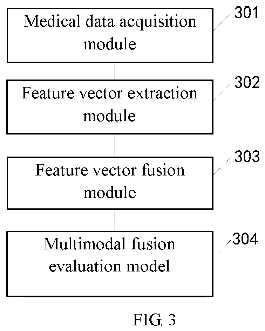

An example of the present disclosure further provides an evaluation device for multimodal medical data fusion, as shown in FIG 3, the device includes:

> a medical data acquisition module 301, configured to acquire to-be-evaluated medical data in multiple modes of a target object;
>
> an feature vector extraction module 302, configured to perform feature extraction on to-be-evaluated medical data in each mode to obtain a plurality of feature vectors;
>
> an feature vector fusion module 303, configured to fuse the a plurality of feature vectors to obtain a fusion feature vector; and
>
> a multimodal fusion evaluation module 304, configured to input the fusion feature vector into a pre-trained multimodal fusion evaluation model to obtain an evaluation result of the to-be-evaluated medical data in multiple modes output by the pre-trained multimodal fusion evaluation model.

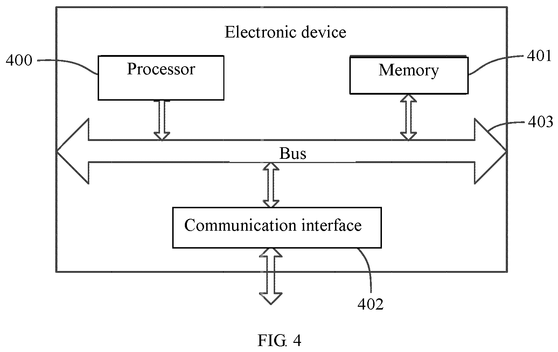

An example of the present disclosure further provides an electronic device, where the structure thereof is shown as FIG.4, including:

A processor 400 and a memory 401, further probably including a communication interface 402 and a communication bus 403. Where, the processor 400, the communication interface 402 and the memory 401 may complete communication with each other through the communication bus 403. The communication interface 402 may be used for information transmission. The processor 400 may call logic instructions in the memory 401 to execute an evaluation method of multimodal medical data fusion in the examples.

Besides, If implemented in a form of software functional units, and sold or used as independent products, the logic instructions in the memory 401 may be stored in a computer-readable storage medium.

As a computer-readable storage medium, the memory 401 may be applied to storage software programs and computer executable programs, such as program instructions/modules corresponding to the method in the examples of the present disclosure. The processor 400 performs function application and data processing by running the program instructions/modules stored in the memory 401, so as to implement the evaluation method for multimodal medical data fusion in the method examples.

The memory 401 may include a program storage area and a data storage area, where the program storage area may store an operating system, at least one application program required by a function, etc.; and the data storage area may store data created on the basis of usage of an electronic device, etc. In addition, the memory 401 may include a high-speed random access memory, and may further include a non-volatile memory.

An example of the present disclosure further provides a computer-readable storage medium, which stores computer-executable instructions that are set to implement the evaluation method for multimodal medical data fusion.

An example of the present disclosure provides a computer program product, which includes a computer program stored on a computer-readable storage medium. The computer program includes program instructions. When the program instructions are executed by a computer, the computer is enabled to implement the evaluation method for multimodal medical data fusion.

The computer-readable storage medium may be a transient computer-readable storage medium or a non-transient computer-readable storage medium.

An evaluation method and device for multimodal medical data fusion, as well as a device and a storage medium provided by examples of the present disclosure adopt a 3D convolution network (3DCNN) technology to fuse multi-view images, and extract fusion features of macro-view images, near-view images and micro-view images of rectal cancer under endoscope. In view of the traditional machine learning prediction model, input requires a standardized data format, and performance may be affected greatly if the input requirement is not met; while the multimodal fusion evaluation model based on artificial intelligence proposed in the present application not only has excellent performance, but also has self-attention weight, may still have relatively excellent performance in the case of partial missing data (at least three modal data of four modal data should be input) by relying on self-perception ability, and may output an evaluation result quickly and accurately, thereby being closer to the clinical use scenario. The model may assist in accurate evaluation of the disease remission degree of a target object at the pathological level, thus improving judgment accuracy and reducing medical risks of the target object.

Technical solutions of examples of the present disclosure may be embodied in a form of a software product; and the computer software product is stored in a storage medium and includes at least one instruction which is used to enable a computer device (which may be a personal computer, a server, or a network device, etc.) to execute all or part of steps of the method described in the examples of the present disclosure. The foregoing storage medium may be a non-transient storage medium, including: USB flash disk, removable hard disk, read-only memory (ROM), random access memory (RAM), magnetic disk or optical disk and other media that may store program codes, and may also be a transient storage medium.

The above description and drawings fully illustrate the examples of the present disclosure to enable those skilled in the art to practice them. Other examples may include structural, logical, electrical, procedural and other changes. The examples represent only possible changes. Unless explicitly required, individual components and functions are optional, and the order of operation may be changed. The parts and features of some examples may be included in or replace those of other examples. When used in this application, although the terms "first" and "second" may be used in this application to describe various elements, these elements should not be limited by these terms. These terms are only used to distinguish one component from another. For example, without changing the meaning of the description, the first component may be called the second component, and similarly, the second component may be called the first component, as long as all the "first components" that appear are renamed and all the "second components" that appear are renamed. The first component and the second component are both components, but they may not be the same. Moreover, the words used in this application are only used to describe the examples and are not used to limit the claims. As used in the description of examples and claims, unless the context clearly indicates, "a", "an" and "the" in the singular form are intended to also include the plural form. Similarly, the term "and/or" as used in the present application refers to any or all possible combinations of one or more associated listed items. In addition, when used in this application, the term "comprise" and its variant "comprises" and/or "comprising" refer to the existence of the stated features, whole, steps, operations, elements, and/or components, but do not exclude the existence or addition of one or more other features, whole, steps, operations, elements, components, and/or groupings thereof. In the absence of more limitations, an element defined by "include a..." does not exclude other same elements existing in the process, method or device including the element. In the specification, each example may focus on the differences from the other examples, and the same or similar parts between the various examples may refer to each other. For the method and product disclosed in the examples, if they correspond to the method section disclosed in the examples, the description of the method section may be referred to for relevance.

Those skilled in the art may realize that in combination with units and algorithmic steps of each example described in the examples of the present disclosure, the examples of the present disclosure can be realized in the form of electronic hardware or a combination of computer software and electronic hardware. Whether these functions are performed in hardware or software may depend on the specific application and design constraints of the technical solution. Those skilled in the art may use different methods for each specific application to realize the described functions. Those skilled in the art may clearly understand that for the convenience and conciseness of the description, the working process of the system, device and unit described above may refer to the corresponding process in the previous method examples, and will not be described in detail.

In the disclosed examples, the disclosed methods and products (including but not limited to devices, equipment, etc.) may be implemented in other ways. For example, the above-described apparatus examples are merely schematic, for example, the division of the units may be merely a logical function division, and there may be other division manners in actual realization, for example, a plurality of units or components may be combined or integrated into another system, or some features may be ignored or not executed. Besides, the mutual coupling or direct coupling or communication connection shown or discussed may be indirect coupling or communication connection of the apparatuses or units through some interfaces, and may be electrical, mechanical or in other forms. The units described as separate components may or may not be physically separated, and the components displayed as units may or may not be physical units, that is, the components may be located in one place, or may be distributed to a plurality of network units. Part or all of the units may be selected according to actual needs to realize the examples. In addition, various functional units in the examples of the present disclosure may be integrated in one processing unit, or each unit may exist individually and physically, or two or more units may be integrated in one unit.

The flowcharts and block diagrams in the drawings show the possibly implemented architecture, functions and operations of the systems, methods and computer program products in the examples of the present disclosure. In this regard, each block in the flowcharts or block diagrams may represent a module, program segment, or part of a code, and the module, program segment, or part of the code includes at least one executable instruction for implementing the specified logical functions. In some alternative implementations, the functions annotated in the blocks may also occur in a different order from the order annotated in the drawings. For example, two consecutive blocks may actually be executed substantially in parallel, or they may sometimes be executed in a reverse order, which depends on the functions involved. In the description corresponding to the flowcharts and block diagrams in the drawings, the operations or steps corresponding to different blocks may also occur in a different order from the sequence disclosed in the description, and sometimes there is no specific sequence between different operations or steps. For example, two consecutive blocks may actually be executed substantially in parallel, or they may sometimes be executed in a reverse order, which depends on the functions involved. Each block in the block diagrams and/or flowcharts, and a combination of the blocks in the block diagrams and/or flowcharts, may be implemented by a dedicated hardware-based system that performs the specified functions or actions, or may be implemented by a combination of dedicated hardware and computer instructions.

**Claims**

1.  A computer-implemented evaluation method for multimodal medical data fusion, the method comprising:

acquiring to-be-evaluated medical data in multiple modes of a target object;

performing feature extraction on the to-be-evaluated medical data in each mode to obtain a plurality of feature vectors;

fusing the a plurality of feature vectors to obtain a fusion feature vector; and

inputting the fusion feature vector into a pre-trained multimodal fusion evaluation model to obtain an evaluation result of the to-be-evaluated medical data in multiple modes output by the pre-trained multimodal fusion evaluation model;

wherein the inputting the fusion feature vector into a pre-trained multimodal fusion evaluation model to obtain an evaluation result of the to-be-evaluated medical data in multiple modes output by the pre-trained multimodal fusion evaluation model comprises:

splicing each feature vector in the fusion feature vector horizontally to obtain a first matrix W(In) of feature vector, and coding position of the first matrix W(In) of feature vector by a first function to obtain a second matrix W(P) of feature vector, wherein the formula is as follows:

$$p(t) = \begin{cases} sin\left(\dfrac{pos}{10000^{2i/d}}\right), & i \% 2 = 0 \\ cos\left(\dfrac{pos}{10000^{2i/d}}\right), & i \% 2 = 1 \end{cases}$$

wherein, t represents a sub-vector in the first matrix W(In) of feature vector; p(t) represents a coding result corresponding to the t value; pos indicates which feature vector the vector t belongs to; i represents a sequence number of the vector t in the first matrix W(In) of feature vector; and d represents the number of horizontal dimensions of the first matrix W(In) of feature vector;

inputting the second matrix W(P) of feature vector into a second function, and calculating and obtaining a high-dimensional feature representation matrix W(M) on a subspace wherein the formula is as follows:

$$W(M) = Concat(F(1), F(2), \ldots, F(i)) \bullet W^0 ;$$

$$F(i) = softmax\left(\frac{Q(x)\ K^T(x)}{\sqrt{d(x)}}\right) \bullet V$$

wherein, a CONCAT function represents the second function, and F(1), F(2)... F(i) represents the ith feature sub-vector in the second matrix W(P) of feature vector is conducted the formula F calculation; $W^0$ represents transposition of the first matrix W(In) of feature vector;

x in F(i) represents the ith feature sub-vector in the input second matrix W(P) of feature vector; Q, K and V represent the linear sensing layer of parameter n of the hidden layer of the multimodal fusion evaluation model; and Q (x) represents linear regression of x; and

coding the feature vector of each image through an encoder of the multimodal fusion evaluation model, inputting output W(Out) of the encoder to a linear regression layer, converting W(Out) to a low-dimensional feature representation matrix through the linear regression layer, and finally outputting an evaluation result through operation of a softmax function; the final result is an evaluation result of complete or incomplete disease remission of the target object, as well as probability corresponding to the evaluation result;

wherein the acquiring the to-be-evaluated medical data in multiple modes of the target object comprises at least three of the following:

acquiring a rectal cancer image data set of the target object as first modal data, wherein the rectal cancer image data set at least comprises a macro-view image, a near-view image and a micro-view image determined according to a tumor region or a regressing tumor region;

acquiring a rectal cancer magnetic resonance image data set of the target object as second modal data, wherein the rectal cancer magnetic resonance image data set comprises initial rectal cancer magnetic resonance image data and target rectal cancer magnetic resonance image data; marking a tumor region or a regressing tumor region in the initial rectal cancer magnetic resonance image data and the target rectal cancer magnetic resonance image data respectively to obtain a plurality of slice images containing the tumor region or the regressing tumor region;

acquiring an initial clinical data set and a target clinical data set of the target object as a third modal data, wherein the initial clinical data set and the target clinical data set at least comprise personal information and case information of the target object; and

acquiring initial tumor marker information, target tumor marker information, initial blood information and target blood information of the target object as fourth modal data.

2. The method according to claim 1, wherein the rectal cancer image data set of the target object as first modal data is acquired through an endoscope; and wherein the tumor region or regressing tumor region in the initial rectal cancer magnetic resonance image data and the target rectal cancer magnetic resonance image data are marked respectively through automatic or manual annotation to obtain the plurality of slice images containing the tumor region or the regressing tumor region, wherein the initial rectal cancer magnetic resonance image data may be data of the target object before treatment, and the target rectal cancer magnetic resonance image data may be data of the target object after treatment.

3. The method according to claim 1 or 2, wherein the performing feature extraction on to-be-evaluated medical data in each mode to obtain a plurality of feature vectors comprises:

inputting the first modal data and the second modal data to a pre-trained neural network model respectively;

performing matrix connection on medical images in the first modal data and the second modal data respectively througha hard-wired layerof the neural network model;

performing convolution calculation and maximum pooling operation on a medical image after the matrix connection through alpha 3D convolution modules of the neural network model to extract high-dimensional feature maps; and

converting a high-dimensional feature map extracted from the last 3D convolution kernel into a one-dimensional feature vector through beta up-sampling modules and a full-connection layer of the neural network model to obtain the first feature vector and a second feature vector respectively.

4. The method according to claim 1 or 2, wherein the performing feature extraction on to-be-evaluated medical data in each mode to obtain a plurality of feature vectors comprises:

mapping text description features in the third modal data and the fourth modal data into corresponding numerical features; and

mapping the numerical features into a two-dimensional matrix to obtain a third feature vector and a fourth feature vector respectively.

5. The method according to claim 3, wherein a training process of the neural network model comprises:

inputting acquired preset to-be-evaluated medical data as a training sample into a corresponding initial neural network model to enable the initial neural network model to output a corresponding initial feature vector;

if the initial feature vector meets preset requirements, determining the initial neural network model to be successfully trained to obtain the pre-trained neural network model;

if the initial feature vector does not meet the preset requirements, continuing to train the initial neural network model by adjusting a loss parameter in the initial neural network model until the loss parameter is fitted to reach a preset loss parameter threshold so as obtain the pre-trained neural network model.

6. The method according to claim 1 or 2, wherein a cross-entropy loss function is used for parameter back-propagation and update during training of the multimodal fusion evaluation model until the cross-entropy loss function is fitted.

7. An evaluation device for multimodal medical data fusion, wherein the device comprises:

a medical data acquisition module, configured to acquire to-be-evaluated medical data in multiple modes of a target object;

an feature vector extraction module, configured to perform feature extraction on the to-be-evaluated medical data in each mode to obtain a plurality of feature vectors;

an feature vector fusion module, configured to fuse the a plurality of feature vectors to obtain a fusion feature vector; and

a multimodal fusion evaluation module, configured to input the fusion feature vector into a pre-trained multimodal fusion evaluation model to obtain an evaluation result of the to-be-evaluated medical data in multiple modes output by the pre-trained multimodal fusion evaluation model, and specifically configured to:

splice each feature vector in the fusion feature vector horizontally to obtain a first matrix W(In) of feature vector, and code position of the first matrix W(In) of feature vector by a first function to obtain a second matrix W(P) of feature vector, wherein the formula is as follows:

$$p(t) = \begin{cases} sin\left(\dfrac{pos}{10000^{2i/d}}\right), & i\%2=0 \\ cos\left(\dfrac{pos}{10000^{2i/d}}\right), & i\%2=1 \end{cases}$$

wherein, t represents a sub-vector in the first matrix W(In) of feature vector; p(t) represents a coding result corresponding to the t value; pos indicates which feature vector the vector t belongs to; i represents a sequence number of the vector t in the first matrix W(In) of feature vector; and d represents the number of horizontal dimensions of the first matrix W(In) of feature vector;

input the second matrix W(P) of feature vector into a second function, and calculate and obtain a high-dimensional feature representation matrix W(M) on a subspace, wherein the formula is as follows:

$$W(M) = Concat(F(1), F(2), \ldots, F(i)) \cdot W^0;$$

$$F(i) = softmax\left(\dfrac{Q(x)\ K^T(x)}{\sqrt{d(x)}}\right) \cdot V$$

wherein, a CONCAT function represents the second function, and F(1), F(2)... F(i) represents the ith feature sub-vector in the second matrix W(P) of feature vector is conducted the formula F calculation; $W^0$ represents transposition of the first matrix W(In) of feature vector;

x in F(i) represents the ith feature sub-vector in the input second matrix W(P) of feature vector; Q, K and V represent the linear sensing layer of parameter n of the hidden layer of the multimodal fusion evaluation model; and Q (x) represents linear regression of x; and

code the feature vector of each image through an encoder of the multimodal fusion evaluation model, input output W(Out) of the encoder to a linear regression layer, convert W(Out) to a low-dimensional feature representation matrix through the linear regression layer, and finally output an evaluation result through operation of a softmax function; the final result is an evaluation result of complete or incomplete disease remission of the target object, as well as probability corresponding to the evaluation result;

wherein the acquiring the to-be-evaluated medical data in multiple modes of the target object comprises at least three of the following:

acquiring a rectal cancer image data set of the target object as first modal data, wherein the rectal cancer image data set at least comprises a macro-view image, a near-view image and a micro-view image determined according to a tumor region or a regressing tumor region;

acquiring a rectal cancer magnetic resonance image data set of the target object as second modal data, wherein the rectal cancer magnetic resonance image data set comprises initial rectal cancer magnetic resonance image data and target rectal cancer magnetic resonance image data; marking a tumor region or a regressing tumor region in the initial rectal cancer magnetic resonance image data and the target rectal cancer magnetic resonance image data respectively to obtain a plurality of slice images containing the tumor region or the regressing tumor region;

acquiring an initial clinical data set and a target clinical data set of the target object as a third modal data, wherein the initial clinical data set and the target clinical data set at least comprise personal information and case information of the target object; and

acquiring initial tumor marker information, target tumor marker information, initial blood information and target blood information of the target object as fourth modal data.

8. An electronic device, wherein the device comprises a processor, a communication interface, a memory and a communication bus, wherein the processor, the communication interface and the memory complete communication with each other through the communication bus;

a memory, configured to store a computer program; and
a processor, configured to implement the method steps according to any one of claims 1 to 6 when executing the program stored on the memory.

9. A computer-readable storage medium, where a computer program is stored, wherein method steps according to any one of claims 1 to 6 are implemented when the computer program is executed by a processor.

**Patentansprüche**

1. Computerimplementiertes Bewertungsverfahren für multimodale medizinische Datenfusion, das Verfahren umfassend:

Erfassen von zu bewertenden medizinischen Daten in mehreren Modi eines Zielobjekts;
Durchführen von Merkmalsextraktion an den zu bewertenden medizinischen Daten in jedem Modus, um eine Vielzahl von Merkmalsvektoren zu erlangen;
Verschmelzen der Vielzahl von Merkmalsvektoren, um einen Fusionsmerkmalsvektor zu erlangen; und
Eingeben des Fusionsmerkmalsvektors in ein vortrainiertes multimodales Fusionsbewertungsmodell, um ein Bewertungsresultat der zu bewertenden medizinischen Daten in mehreren Modi zu erlangen, das von dem vortrainierten multimodalen Fusionsbewertungsmodell ausgegeben wird;
wobei das Eingeben des Fusionsmerkmalsvektors in ein vortrainiertes multimodales Fusionsbewertungsmodell, um ein Bewertungsresultat der zu bewertenden medizinischen Daten in mehreren Modi zu erlangen, das von dem vortrainierten multimodalen Fusionsbewertungsmodell ausgegeben wird, Folgendes umfasst:

horizontales Zusammenfügen von jedem Merkmalsvektor in dem Fusionsmerkmalsvektor, um eine erste Matrix W(In) des Merkmalsvektors zu erlangen, und Kodieren einer Position der ersten Matrix W(In) des Merkmalsvektors durch eine erste Funktion, um eine zweite Matrix W(P) des Merkmalsvektors zu erlangen, wobei die Formel wie folgt ist:

$$p(t) = \begin{cases} sin\left(\dfrac{pos}{10000^{2i/d}}\right), & i\ \%\ 2 = 0 \\ cos\left(\dfrac{pos}{10000^{2i/d}}\right), & i\ \%\ 2 = 1 \end{cases}$$

wobei t einen Untervektor in der ersten Matrix W(In) des Merkmalsvektors darstellt; p(t) ein Kodierungsresultat darstellt, das dem t-Wert entspricht; pos angibt, zu welchem Merkmalsvektor der Vektor t gehört; i eine Sequenznummer des Vektors t in der ersten Matrix W(In) des Merkmalsvektors darstellt; und d die Anzahl an horizontalen Dimensionen der ersten Matrix W(In) des Merkmalsvektors darstellt;
Eingeben der zweiten Matrix W(P) des Merkmalsvektors in eine zweite Funktion und Berechnen und Erlangen einer hochdimensionalen Merkmalsdarstellungsmatrix W(M) auf einem Unterraum, wobei die Formel wie folgt ist:

$$W(M) = Concat\left(F(1), F(2), \ldots, F(i)\right) \cdot W^0;$$

$$F(i) = softmax\left(\dfrac{Q(x)\ K^T(x)}{\sqrt{d(x)}}\right) \cdot V$$

wobei eine CONCAT-Funktion die zweite Funktion darstellt, und F(1), F(2)... F(i) den i-ten Merkmalsuntervektor in der zweiten Matrix W(P) des Merkmalsvektors, für den die Formel F berechnet wird; $W^0$ eine Transposition der ersten Matrix W(In) des Merkmalsvektors darstellt;
x in F(i) den i-ten Merkmalsuntervektor in der zweiten Eingangsmatrix W(P) des Merkmalsvektors darstellt;
Q, K und V die lineare Erfassungsschicht von Parameter n der verborgenen Schicht des multimodalen Fusionsbewertungsmodells darstellen; und Q (x) die lineare Regression von x darstellt; und

Kodieren des Merkmalsvektors von jedem Bild durch einen Kodierer des multimodalen Fusionsbewertungsmodells, Eingeben eines Ausgangs W(Out) des Kodierers in eine lineare Regressionsschicht, Umwandeln von W(Out) in eine niedrigdimensionale Merkmalsdarstellungsmatrix durch die lineare Regressionsschicht, und schließlich Ausgeben eines Bewertungsresultats durch die Operation einer Softmax-Funktion; wobei das endgültige Resultat ein Bewertungsresultat der vollständigen oder unvollständigen Krankheitsremission des Zielobjekts sowie eine dem Bewertungsresultat entsprechende Wahrscheinlichkeit ist;

wobei das Erfassen der zu bewertenden medizinischen Daten in mehreren Modi des Zielobjekts mindestens drei der Folgenden umfasst:

Erfassen eines Rektumkarzinom-Bilddatensatzes des Zielobjekts als erste Modaldaten, wobei der Rektumkarzinom-Bilddatensatz mindestens ein Makroansichtsbild, ein Nahansichtsbild und ein Mikroansichtsbild umfasst, die gemäß einem Tumorbereich oder einem sich zurückbildenden Tumorbereich bestimmt werden;

Erfassen eines Rektumkarzinom-Magnetresonanzbilddatensatzes des Zielobjekts als zweite Modaldaten, wobei der Rektumkarzinom-Magnetresonanzbilddatensatz anfängliche Rektumkarzinom-Magnetresonanzbilddaten und Ziel-Rektumkarzinom-Magnetresonanzbilddaten umfasst; Markieren eines Tumorbereichs oder eines sich zurückbildenden Tumorbereichs jeweils in den anfänglichen Rektumkarzinom-Magnetresonanzbilddaten und den Ziel-Rektumkarzinom-Magnetresonanzbilddaten, um eine Vielzahl von Schichtbildern zu erlangen, die den Tumorbereich oder den sich zurückbildenden Tumorbereich enthalten;

Erfassen eines anfänglichen klinischen Datensatzes und eines klinischen Zieldatensatzes des Zielobjekts als dritte Modaldaten, wobei der anfängliche klinische Datensatz und der klinische Zieldatensatz zumindest personenbezogene Informationen und Fallinformationen des Zielobjekts umfassen; und

Erfassen von anfänglichen Tumormarkerinformationen, Ziel-Tumormarkerinformationen, anfänglichen Blutinformationen und Ziel-Blutinformationen des Zielobjekts als vierte Modaldaten.

2. Verfahren nach Anspruch 1, wobei der Rektumkarzinom-Bilddatensatz des Zielobjekts als erste Modaldaten durch ein Endoskop erfasst wird; und wobei der Tumorbereich oder der sich zurückbildende Tumorbereich in den anfänglichen Rektumkarzinom-Magnetresonanzbilddaten und den Ziel-Rektumkarzinom-Magnetresonanzbilddaten jeweils durch automatische oder manuelle Beschriftung markiert werden, um die Vielzahl von Schichtbildern zu erlangen, die den Tumorbereich oder den sich zurückbildenden Tumorbereich enthalten, wobei die anfänglichen Rektumkarzinom-Magnetresonanzbilddaten Daten des Zielobjekts vor der Behandlung sein können und die Ziel-Rektumkarzinom-Magnetresonanzbilddaten Daten des Zielobjekts nach der Behandlung sein können.

3. Verfahren nach Anspruch 1 oder 2, wobei das Durchführen von Merkmalsextraktion an den zu bewertenden medizinischen Daten in jedem Modus zum Erlangen einer Vielzahl von Merkmalsvektoren Folgendes umfasst:

Eingeben der ersten Modaldaten und der zweiten Modaldaten jeweils in ein vortrainiertes neuronales Netzmodell;

Durchführen von Matrixverbindung jeweils an medizinischen Bildern in den ersten modalen Daten und den zweiten modalen Daten durch eine fest verdrahtete Schicht des neuronalen Netzmodells;

Durchführen von Faltungsberechnung und maximaler Pooling-Operation an einem medizinischen Bild nach der Matrixverbindung durch Alpha-3D-Faltungsmodule des neuronalen Netzmodells, um hochdimensionale Merkmalskarten zu extrahieren; und

Umwandeln einer hochdimensionalen Merkmalskarte, die aus dem letzten 3D-Faltungskernel extrahiert wird, in einen eindimensionalen Merkmalsvektor durch Beta-Up-Sampling-Module und eine Vollverbindungsschicht des neuronalen Netzmodells, um jeweils den ersten Merkmalsvektor und einen zweiten Merkmalsvektor zu erlangen.

4. Verfahren nach Anspruch 1 oder 2, wobei das Durchführen von Merkmalsextraktion an den zu bewertenden medizinischen Daten in jedem Modus zum Erlangen einer Vielzahl von Merkmalsvektoren Folgendes umfasst:

Mappen von Textbeschreibungsmerkmalen in den dritten Modaldaten und den vierten Modaldaten in entsprechende numerische Merkmale; und

Mappen der numerischen Merkmale in eine zweidimensionale Matrix, um jeweils einen dritten Merkmalsvektor und einen vierten Merkmalsvektor zu erlangen.

5. Verfahren nach Anspruch 3, wobei ein Trainingsprozess des neuronalen Netzmodells Folgendes umfasst:

Eingeben erfasster, voreingestellter, zu bewertender medizinischer Daten als Trainingsmuster in ein entsprechendes anfängliches neuronales Netzmodell, um zu ermöglichen, dass das anfängliche neuronale Netzmodell einen entsprechenden anfänglichen Merkmalsvektor ausgibt;

wenn der anfängliche Merkmalsvektor die voreingestellten Anforderungen erfüllt, Bestimmen des anfänglichen neuronalen Netzmodells, das erfolgreich trainiert werden soll, um das vortrainierte neuronale Netzmodell zu erlangen;

wenn der anfängliche Merkmalsvektor nicht den voreingestellten Anforderungen entspricht, Fortsetzen eines Trainierens des anfänglichen neuronalen Netzmodells durch Anpassen eines Verlustparameters in dem anfänglichen neuronalen Netzmodell, bis der Verlustparameter angepasst ist, um einen voreingestellten Schwellenwert des Verlustparameters zu erreichen, um das vortrainierte neuronale Netzmodell zu erlangen.

6. Verfahren nach Anspruch 1 oder 2, wobei eine Kreuzentropie-Verlustfunktion für die Parameterrückentwicklung und -aktualisierung während eines Trainierens des multimodalen Fusionsbewertungsmodells verwendet wird, bis die Kreuzentropie-Verlustfunktion angepasst ist.

7. Bewertungsvorrichtung für multimodale medizinische Datenfusion, wobei die Vorrichtung Folgendes umfasst:

ein medizinisches Datenerfassungsmodul, das konfiguriert ist, um die zu bewertenden medizinischen Daten eines Zielobjekts in mehreren Modi zu erfassen;

ein Merkmalsvektorextraktionsmodul, das konfiguriert ist, um eine Merkmalsextraktion an den zu bewertenden medizinischen Daten in jedem Modus durchzuführen, um eine Vielzahl von Merkmalsvektoren zu erlangen;

ein Merkmalsvektorfusionsmodul, das konfiguriert ist, um eine Vielzahl von Merkmalsvektoren zu fusionieren, um einen Fusionsmerkmalsvektor zu erlangen; und

ein multimodales Fusionsbewertungsmodul, das konfiguriert ist, um den Fusionsmerkmalvektor in ein vortrainiertes multimodales Fusionsauswertungsmodell einzugeben, um ein Bewertungsresultat der zu bewertenden medizinischen Daten in mehreren Modi zu erlangen, das von dem vortrainierten multimodalen Fusionsauswertungsmodell ausgegeben wird, und das speziell zu Folgendem konfiguriert ist:

horizontales Zusammenfügen von jedem Merkmalsvektor in dem Fusionsmerkmalsvektor, um eine erste Matrix W(In) des Merkmalsvektors zu erlangen, und Kodieren einer Position der ersten Matrix W(In) des Merkmalsvektors durch eine erste Funktion, um eine zweite Matrix W(P) des Merkmalsvektors zu erlangen, wobei die Formel wie folgt ist:

$$p(t) = \begin{cases} sin\left(\dfrac{pos}{10000^{2i/d}}\right), & i\,\%\,2 = 0 \\ cos\left(\dfrac{pos}{10000^{2i/d}}\right), & i\,\%\,2 = 1 \end{cases}$$

wobei t einen Untervektor in der ersten Matrix W(In) des Merkmalsvektors darstellt; p(t) ein Kodierungsresultat darstellt, das dem t-Wert entspricht; pos angibt, zu welchem Merkmalsvektor der Vektor t gehört; i eine Sequenznummer des Vektors t in der ersten Matrix W(In) des Merkmalsvektors darstellt; und d die Anzahl an horizontalen Dimensionen der ersten Matrix W(In) des Merkmalsvektors darstellt;

Eingeben der zweiten Matrix W(P) des Merkmalsvektors in eine zweite Funktion und Berechnen und Erlangen einer hochdimensionalen Merkmalsdarstellungsmatrix W(M) auf einem Unterraum, wobei die Formel wie folgt ist:

$$W(M) = Concat(F(1), F(2), \ldots, F(i)) \cdot W^{0};$$

$$F(i) = softmax\left(\frac{Q(x)\ K^{T}(x)}{\sqrt{d(x)}}\right) \cdot V$$

wobei eine CONCAT-Funktion die zweite Funktion darstellt, und F(1), F(2)... F(i) den i-ten Merkmalsuntervektor in der zweiten Matrix W(P) des Merkmalsvektors, für den die Formel F berechnet wird; $W^{0}$ eine Transposition der ersten Matrix W(In) des Merkmalsvektors darstellt;

x in F(i) den i-ten Merkmalsuntervektor in der zweiten Eingangsmatrix W(P) des Merkmalsvektors darstellt;

Q, K und V die lineare Erfassungsschicht von Parameter n der verborgenen Schicht des multimodalen Fusionsbewertungsmodells darstellen; und Q (x) die lineare Regression von x darstellt; und

Kodieren des Merkmalsvektors von jedem Bild durch einen Kodierer des multimodalen Fusionsbewertungsmodells, Eingeben eines Ausgangs W(Out) des Kodierers in eine lineare Regressionsschicht, Umwandeln von W(Out) in eine niedrigdimensionale Merkmalsdarstellungsmatrix durch die lineare Regressionsschicht, und schließlich Ausgeben eines Bewertungsresultats durch die Operation einer Softmax-Funktion; wobei das endgültige Resultat ein Bewertungsresultat der vollständigen oder unvollständigen Krankheitsremission des Zielobjekts sowie eine dem Bewertungsresultat entsprechende Wahrscheinlichkeit ist;

wobei das Erfassen der zu bewertenden medizinischen Daten in mehreren Modi des Zielobjekts mindestens drei der Folgenden umfasst:

Erfassen eines Rektumkarzinom-Bilddatensatzes des Zielobjekts als erste Modaldaten, wobei der Rektumkarzinom-Bilddatensatz mindestens ein Makroansichtsbild, ein Nahansichtsbild und ein Mikroansichtsbild umfasst, die gemäß einem Tumorbereich oder einem sich zurückbildenden Tumorbereich bestimmt werden;

Erfassen eines Rektumkarzinom-Magnetresonanzbilddatensatzes des Zielobjekts als zweite Modaldaten, wobei der Rektumkarzinom-Magnetresonanzbilddatensatz anfängliche Rektumkarzinom-Magnetresonanzbilddaten und Ziel-Rektumkarzinom-Magnetresonanzbilddaten umfasst; Markieren eines Tumorbereichs oder eines sich zurückbildenden Tumorbereichs jeweils in den anfänglichen Rektumkarzinom-Magnetresonanzbilddaten und den Ziel-Rektumkarzinom-Magnetresonanzbilddaten, um eine Vielzahl von Schichtbildern zu erlangen, die den Tumorbereich oder den sich zurückbildenden Tumorbereich enthalten;

Erfassen eines anfänglichen klinischen Datensatzes und eines klinischen Zieldatensatzes des Zielobjekts als dritte Modaldaten, wobei der anfängliche klinische Datensatz und der klinische Zieldatensatz zumindest personenbezogene Informationen und Fallinformationen des Zielobjekts umfassen; und Erfassen von anfänglichen Tumormarkerinformationen, Ziel-Tumormarkerinformationen, anfänglichen Blutinformationen und Ziel-Blutinformationen des Zielobjekts als vierte Modaldaten.

8. Elektronische Vorrichtung, wobei die Vorrichtung einen Prozessor, eine Kommunikationsschnittstelle, einen Speicher und einen Kommunikationsbus umfasst, wobei der Prozessor, die Kommunikationsschnittstelle und der Speicher die Kommunikation miteinander über den Kommunikationsbus abschließen;

einen Speicher, der zum Speichern eines Computerprogramms konfiguriert ist; und
einen Prozessor, der konfiguriert ist, um die Verfahrensschritte nach einem der Ansprüche 1 bis 6 durchzuführen, wenn er das in dem Speicher gespeicherte Programm ausführt.

9. Computerlesbares Speichermedium, auf dem ein Computerprogramm gespeichert ist, wobei Verfahrensschritte nach einem der Ansprüche 1 bis 6 implementiert werden, wenn das Computerprogramm von einem Prozessor ausgeführt wird.

**Revendications**

1. - Procédé d'évaluation mis en œuvre par ordinateur pour une fusion de données médicales multimodale, le procédé comprenant :

acquérir des données médicales à évaluer dans de multiples modes d'un objet cible ;
effectuer une extraction de caractéristiques sur les données médicales à évaluer dans chaque mode pour obtenir une pluralité de vecteurs de caractéristiques ;
fusionner la pluralité de vecteurs de caractéristiques pour obtenir un vecteur de caractéristiques de fusion ; et
entrer le vecteur de caractéristiques de fusion dans un modèle d'évaluation de fusion multimodale pré-entraîné afin d'obtenir un résultat d'évaluation des données médicales à évaluer dans de multiples modes délivré par le modèle d'évaluation de fusion multimodale pré-entraîné ;
dans lequel l'entrée du vecteur de caractéristiques de fusion dans un modèle d'évaluation de fusion multimodale pré-entraîné afin d'obtenir un résultat d'évaluation des données médicales à évaluer dans de multiples modes délivré par le modèle d'évaluation de fusion multimodale pré-entraîné comprend :

assembler chaque vecteur de caractéristiques dans le vecteur de caractéristiques de fusion horizontalement pour obtenir une première matrice W(In) de vecteur de caractéristiques, et coder une position de la première matrice W(In) de vecteur de caractéristiques par une première fonction pour obtenir une seconde matrice W(P) de vecteur de caractéristiques, la formule étant la suivante :

$$p(t) = \begin{cases} sin\left(\dfrac{pos}{10000^{2i/d}}\right), & i \% 2 = 0 \\ cos\left(\dfrac{pos}{10000^{2i/d}}\right), & i \% 2 = 1 \end{cases}$$

où t représente un sous-vecteur dans la première matrice W(In) de vecteur de caractéristiques ; p(t) représente un résultat de codage correspondant à la valeur t ; pos indique à quel vecteur de caractéristiques le vecteur t appartient ; i représente un numéro de séquence du vecteur t dans la première matrice W(In) de vecteur de caractéristiques ; et d représente le nombre de dimensions horizontales de la première matrice W(In) de vecteur de caractéristiques ;

entrer la seconde matrice W(P) de vecteur de caractéristiques dans une seconde fonction, et calculer et obtenir une matrice de représentation de caractéristiques à haute dimension W(M) sur un sous-espace, la formule étant la suivante :

$$W(M) = Concat\left(F(1), F(2), \ldots, F(i)\right) \cdot W^0 ;$$

$$F(i) = softmax\left(\frac{Q(x)\ K^T(x)}{\sqrt{d(x)}}\right) \cdot V$$

où une fonction CONCAT représente la seconde fonction, et F(1), F(2)... F(i) représente le $i^{ème}$ sous-vecteur de caractéristiques dans la seconde matrice W(P) de vecteur de caractéristiques pour lequel est conduit le calcul de la formule F ; $W^0$ représente une transposition de la première matrice W(In) de vecteur de caractéristiques ;

x dans F(i) représente le $i^{ème}$ sous-vecteur de caractéristiques dans la seconde matrice d'entrée W(P) de vecteur de caractéristiques ; Q, K et V représentent la couche de détection linéaire du paramètre n de la couche cachée du modèle d'évaluation de fusion multimodale ; et Q(x) représente une régression linéaire de x ; et

coder le vecteur de caractéristiques de chaque image par l'intermédiaire d'un codeur du modèle d'évaluation de fusion multimodale, entrer la sortie W(Out) du codeur dans une couche de régression linéaire, convertir W(Out) en une matrice de représentation de caractéristiques à faible dimension par l'intermédiaire de la couche de régression linéaire, et enfin délivrer un résultat d'évaluation par l'intermédiaire d'une opération d'une fonction softmax ; le résultat final est un résultat d'évaluation de la rémission complète ou incomplète de la maladie de l'objet cible, ainsi qu'une probabilité correspondant au résultat d'évaluation ;

dans lequel l'acquisition des données médicales à évaluer dans de multiples modes de l'objet cible comprend au moins trois de ce qui suit :

acquérir un ensemble de données d'images de cancer du rectum de l'objet cible en tant que premières données modales, l'ensemble de données d'images de cancer du rectum comprenant au moins une image en macro-vision, une image en vision rapprochée et une image en micro-vision déterminées en fonction d'une région tumorale ou d'une région tumorale en régression ;

acquérir un ensemble de données d'images par résonance magnétique de cancer du rectum de l'objet cible en tant que deuxièmes données modales, l'ensemble de données d'images par résonance magnétique de cancer du rectum comprenant des données initiales d'images par résonance magnétique de cancer du rectum et des données cibles d'images par résonance magnétique de cancer du rectum ; marquer une région tumorale ou une région tumorale en régression dans les données initiales d'images par résonance magnétique de cancer du rectum et les données cibles d'images par résonance magnétique de cancer du rectum respectivement pour obtenir une pluralité d'images en tranches contenant la région tumorale ou la région tumorale en régression ;

acquérir un ensemble de données cliniques initiales et un ensemble de données cliniques cibles de l'objet cible en tant que troisièmes données modales, l'ensemble de données cliniques initiales et l'ensemble de données cliniques cibles comprenant au moins des informations personnelles et des informations de dossier de l'objet cible ; et

acquérir des informations initiales sur le marqueur tumoral, des informations cibles sur le marqueur tumoral, des informations initiales sur le sang et des informations cibles sur le sang de l'objet cible en tant que quatrièmes données modales.

2. - Procédé selon la revendication 1, dans lequel l'ensemble de données d'images de cancer du rectum de l'objet cible en tant que premières données modales est acquis par un endoscope ; et dans lequel la région tumorale ou la région tumorale en régression dans les données initiales d'images par résonance magnétique de cancer du rectum et les données cibles d'images par résonance magnétique de cancer du rectum sont marquées respectivement par annotation automatique ou manuelle pour obtenir la pluralité d'images en tranches contenant la région tumorale ou la région tumorale en régression, les données initiales d'images par résonance magnétique de cancer du rectum pouvant être des données de l'objet cible avant traitement, et les données cibles d'images par résonance magnétique de cancer du rectum pouvant être des données de l'objet cible après traitement.

3. - Procédé selon la revendication 1 ou 2, dans lequel la réalisation d'une extraction de caractéristiques sur des données médicales à évaluer dans chaque mode pour obtenir une pluralité de vecteurs de caractéristiques comprend :

entrer respectivement les premières données modales et les deuxièmes données modales dans un modèle de réseau neuronal pré-entraîné ;
effectuer une connexion matricielle sur des images médicales respectivement dans les premières données modales et les deuxièmes données modales par l'intermédiaire d'une couche câblée du modèle de réseau neuronal ;
effectuer un calcul de convolution et une opération de mise en commun maximale sur une image médicale après la connexion matricielle par des modules de convolution 3D alpha du modèle de réseau neuronal pour extraire des cartes de caractéristiques à haute dimension ; et
convertir une carte de caractéristiques à haute dimension extraite à partir du dernier noyau de convolution 3D en un vecteur de caractéristiques unidimensionnel par l'intermédiaire de modules de sur-échantillonnage bêta et d'une couche de connexion complète du modèle de réseau neuronal pour obtenir respectivement le premier vecteur de caractéristiques et un deuxième vecteur de caractéristiques.

4. - Procédé selon la revendication 1 ou 2, dans lequel la réalisation d'une extraction de caractéristiques sur des données médicales à évaluer dans chaque mode pour obtenir une pluralité de vecteurs de caractéristiques comprend :

mapper des caractéristiques de description de texte dans les troisièmes données modales et les quatrièmes données modales dans des caractéristiques numériques correspondantes ; et
mapper les caractéristiques numériques dans une matrice à deux dimensions pour obtenir respectivement un troisième vecteur de caractéristiques et un quatrième vecteur de caractéristiques.

5. - Procédé selon la revendication 3, dans lequel un processus d'entraînement du modèle de réseau neuronal comprend :

entrer des données médicales à évaluer prédéfinies acquises en tant qu'échantillon d'entraînement dans un modèle de réseau neuronal initial correspondant pour permettre au modèle de réseau neuronal initial de délivrer un vecteur de caractéristiques initial correspondant ;
si le vecteur de caractéristiques initial répond à des exigences prédéfinies, déterminer le modèle de réseau neuronal initial comme étant entraîné avec succès pour obtenir le modèle de réseau neuronal pré-entraîné ;
si le vecteur de caractéristiques initial ne répond pas aux exigences prédéfinies, continuer d'entraîner le modèle de réseau neuronal initial par ajustement d'un paramètre de perte dans le modèle de réseau neuronal initial jusqu'à ce que le paramètre de perte soit ajusté pour atteindre un seuil de paramètre de perte prédéfini afin d'obtenir le modèle de réseau neuronal pré-entraîné.

6. - Procédé selon la revendication 1 ou 2, dans lequel une fonction de perte à entropie croisée est utilisée pour la rétropropagation et la mise à jour de paramètre pendant l'entraînement du modèle d'évaluation de fusion multimodale jusqu'à ce que la fonction de perte à entropie croisée soit ajustée.

7.  - Dispositif d'évaluation pour une fusion de données médicales multimodale, dans lequel le dispositif comprend :

un module d'acquisition de données médicales, configuré pour acquérir des données médicales à évaluer dans de multiples modes d'un objet cible ;

un module d'extraction de vecteurs de caractéristiques, configuré pour effectuer une extraction de caractéristiques sur les données médicales à évaluer dans chaque mode afin d'obtenir une pluralité de vecteurs de caractéristiques ;

un module de fusion de vecteurs de caractéristiques, configuré pour fusionner la pluralité de vecteurs de caractéristiques afin d'obtenir un vecteur de caractéristiques de fusion ; et

un module d'évaluation de fusion multimodale, configuré pour entrer le vecteur de caractéristiques de fusion dans un modèle d'évaluation de fusion multimodale pré-entraîné afin d'obtenir un résultat d'évaluation des données médicales à évaluer dans de multiples modes délivré par le modèle d'évaluation de fusion multimodale pré-entraîné, et spécifiquement configuré pour :

assembler chaque vecteur de caractéristiques dans le vecteur de caractéristiques de fusion horizontalement pour obtenir une première matrice W(In) de vecteur de caractéristiques, et coder une position de la première matrice W(In) de vecteur de caractéristiques par une première fonction pour obtenir une seconde matrice W(P) de vecteur de caractéristiques, la formule étant la suivante :

$$p(t) = \begin{cases} sin\left(\dfrac{pos}{10000^{2i/d}}\right), & i\ \%\ 2 = 0 \\ cos\left(\dfrac{pos}{10000^{2i/d}}\right), & i\ \%\ 2 = 1 \end{cases}$$

où t représente un sous-vecteur dans la première matrice W(In) de vecteur de caractéristiques ; p(t) représente un résultat de codage correspondant à la valeur t ; pos indique à quel vecteur de caractéristiques le vecteur t appartient ; i représente un numéro de séquence du vecteur t dans la première matrice W(In) de vecteur de caractéristiques ; et d représente le nombre de dimensions horizontales de la première matrice W(In) de vecteur de caractéristiques ;

entrer la seconde matrice W(P) de vecteur de caractéristiques dans une seconde fonction, et calculer et obtenir une matrice de représentation de caractéristiques à haute dimension W(M) sur un sous-espace, la formule étant la suivante :

$$W(M) = \mathrm{Concat}\left(F(1), F(2), \ldots, F(i)\right) \bullet W^0 ;$$

$$F(i) = \mathrm{softmax}\left(\dfrac{Q(x)\ K^T(x)}{\sqrt{d(x)}}\right) \bullet V$$

où une fonction CONCAT représente la seconde fonction, et F(1), F(2)... F(i) représente le $i^{ème}$ sous-vecteur de caractéristiques dans la seconde matrice W(P) de vecteur de caractéristiques pour lequel est conduit le calcul de la formule F ; $W^0$ représente une transposition de la première matrice W(In) de vecteur de caractéristiques ;

x dans F(i) représente le $i^{ème}$ sous-vecteur de caractéristiques dans la seconde matrice d'entrée W(P) de vecteur de caractéristiques ; Q, K et V représentent la couche de détection linéaire du paramètre n de la couche cachée du modèle d'évaluation de fusion multimodale ; et Q(x) représente une régression linéaire de x ; et

coder le vecteur de caractéristiques de chaque image par un codeur du modèle d'évaluation de fusion multimodale, entrer la sortie W(Out) du codeur dans une couche de régression linéaire, convertir W(Out) en une matrice de représentation de caractéristiques à faible dimension par la couche de régression linéaire, et enfin délivrer un résultat d'évaluation par l'intermédiaire d'une opération d'une fonction softmax ; le résultat final est un résultat d'évaluation d'une rémission complète ou incomplète de la maladie de l'objet cible, ainsi qu'une probabilité correspondant au résultat d'évaluation ;

dans lequel l'acquisition des données médicales à évaluer dans de multiples modes de l'objet cible comprend au moins trois de ce qui suit :

acquérir un ensemble de données d'images de cancer du rectum de l'objet cible en tant que premières données modales, l'ensemble de données d'images de cancer du rectum comprenant au moins une image en macro-vision, une image en vision rapprochée et une image en micro-vision déterminées en fonction d'une région tumorale ou d'une région tumorale en régression ;

acquérir un ensemble de données d'images par résonance magnétique de cancer du rectum de l'objet cible en tant que deuxièmes données modales, l'ensemble de données d'images par résonance magnétique de cancer du rectum comprenant des données initiales d'images par résonance magnétique de cancer du rectum et des données cibles d'images par résonance magnétique de cancer du rectum ; marquer une région tumorale ou une région tumorale en régression respectivement dans les données initiales d'images par résonance magnétique de cancer du rectum et les données cibles d'images par résonance magnétique de cancer du rectum pour obtenir une pluralité d'images en tranches contenant la région tumorale ou la région tumorale en régression ;

acquérir un ensemble de données cliniques initiales et un ensemble de données cliniques cibles de l'objet cible en tant que troisièmes données modales, l'ensemble de données cliniques initiales et l'ensemble de données cliniques cibles comprenant au moins des informations personnelles et des informations de dossier de l'objet cible ; et

acquérir des informations initiales sur le marqueur tumoral, des informations cibles sur le marqueur tumoral, des informations initiales sur le sang et des informations cibles sur le sang de l'objet cible en tant que quatrièmes données modales.

8. - Dispositif électronique, dans lequel le dispositif comprend un processeur, une interface de communication, une mémoire et un bus de communication, dans lequel le processeur, l'interface de communication et la mémoire réalisent une communication entre eux par l'intermédiaire du bus de communication ;

une mémoire, configurée pour stocker un programme informatique ; et

un processeur, configuré pour mettre en œuvre les étapes de procédé selon l'une quelconque des revendications 1 à 6 lors de l'exécution du programme stocké sur la mémoire.

9. - Support de stockage lisible par ordinateur, dans lequel un programme informatique est stocké, dans lequel des étapes de procédé selon l'une quelconque des revendications 1 à 6 sont mises en œuvre lorsque le programme informatique est exécuté par un processeur.

Acquire to-be-evaluated medical data in multiple modes of a target object — S101

↓

Perform feature extraction on to-be-evaluated medical data in each mode to obtain a plurality of feature vectors — S102

↓

Fuse the a plurality of feature vectors to obtain a fusion feature vector — S103

↓

Input the fusion feature vector into a pre-trained multimodal fusion evaluation model to obtain an evaluation result of the to-be-evaluated medical data in multiple modes output by the pre-trained multimodal fusion evaluation model — S104

FIG. 1

FIG 2

Input of first modal

| Macro-view image | Near-view image |

| Micro-view image 1 | Micro-view image 2 |

First neural network model → First feature vector

Input of second modal data

Initial rectal cancer magnetic resonance image data

Target rectal cancer magnetic resonance image data

Second neural network model → Second feature vector

Input of third modal data

Before treatment: age, height, weight, family history of malignant tumor, personal history of tumor, treatment plan, tumor location, tumor differentiation degree, T staging before treatment, N staging before treatment, depth of tumor invasion, and distance from tumor to anal edge

After treatment: age, height, weight, family history of malignant tumor, personal history of tumor, treatment plan, tumor location, tumor differentiation degree, T staging before treatment, N staging before treatment, depth of tumor invasion, and distance from tumor to anal edge

Extraction of third feature vector → Third feature vector

Input of fourth modal data

Before treatment: carbohydrate antigen 125, carbohydrate antigen 153, carbohydrate antigen 199, carcinoembryonic antigen and alpha-fetoprotein, red blood cells, hemoglobin, platelets, platelet volume, white blood cells, neutrophils, lymphocytes, monocytes, C-reactive protein, hypersensitive C-reactive protein, total protein, albumin and prealbumin

After treatment: carbohydrate antigen 125, carbohydrate antigen 153, carbohydrate antigen 199, carcinoembryonic antigen and alpha-fetoprotein, red blood cells, hemoglobin, platelets, platelet volume, white blood cells, neutrophils, lymphocytes, monocytes, C-reactive protein, hypersensitive C-reactive protein, total protein, albumin and prealbumin

Extraction of fourth feature vector → Fourth feature vector

Fusion feature vector

Multimodal fusion evaluation model

Output of evaluation result

Medical data acquisition module 301

Feature vector extraction module 302

Feature vector fusion module 303

Multimodal fusion evaluation model 304

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Predictive models for colorectal cancer recurrence using multi-modal healthcare data. **DANLIANG HO DANLIANG@U NUS EDU et al.** ACM SYMPOSIUM ON EYE TRACKING RESEARCH AND APPLICATIONS. ACMPUB27, 08 April 2021, 204-213